# EUROPEAN PATENT APPLICATION

(11) **EP 3 520 854 A1**
(43) Date of publication of application: **07.08.2019**
(21) Application number: 18170770.4
(22) Date of filing: 04.05.2018
(51) Int. Cl.: A61N 1/05, A61N 1/36

(54) **AMPLITUDE MODULATED STIMULI FOR NEURAL STIMULATION BASED TREATMENTS**

(30) Priority: 02.02.2018 US 201862625348 P
(71) Applicant: BIOTRONIK SE & Co. KG, 12359 Berlin (DE)
(72) Inventor: Raven, Joseph Simon, Tigard, OR 97224 (US)
(74) Representative: Keck, Hans-Georg

(57) **Abstract**

The present invention relates to a method and device for stimulation of nervous tissue, particularly of the spinal cord, wherein an electrical signal comprising pulses is applied to said tissue, wherein a repetition frequency of said pulses is larger than 100 kHz and smaller than 5,000 kHz, and wherein an amplitude of said pulses is modulated such that the electrical signal comprises sub-threshold pulses that do not elicit an action potential, and supra-threshold pulses that elicit an action potential.

## Description

The invention relates to a method and a device for stimulation of nervous tissue, particularly for spinal cord stimulation (SCS).

Chronic and intractable pain is a complex pathophysiological phenomenon that directly impacts the quality of life of patients so afflicted. Spinal cord stimulation (SCS) is an established treatment utilized over the last forty years to treat over 10,000 patients to relieve symptoms associated with chronic neuropathic pain. In particular, the U.S. Food and Drug Administration (FDA) recognizes SCS as an aid in the management of chronic intractable pain of the trunk or limbs, including unilateral or bilateral pain associated with so-called "failed back surgery syndrome," which is generally defined as pain continuing or resuming despite an operative procedure undertaken to correct the cause of the pain.

One specific paresthesia free pain treatment is disclosed US 8,359,102 B2.

Based on the above, it is an objective of the present invention to provide a method and a device for nervous tissue stimulation, particularly for spinal cord stimulation, that allows to use a wider range of stimulation frequencies.

This problem is solved by a method having the features of claim 1 as well as by a device having the features of claim 7. Particular embodiments of the respective aspect of present invention are stated in the corresponding sub claims and are described below.

According to claim 1 a, device for stimulation of nervous tissue is disclosed, wherein the device comprises an implantable pulse generator (IPG) that comprises a plurality of electrodes and is configured to apply an electrical signal via said electrodes to nervous tissue of a patient, such that said electrical signal comprises pulses having a repetition frequency being greater than or equal to 51 kHz and less than or equal to 5,000 kHz, and wherein the implantable pulse generator is further configured to modulate an amplitude of said pulses such that the electrical signal comprises sub-threshold pulses that do not elicit an action potential, and supra-threshold pulses that elicit an action potential.

According to an embodiment of the present invention, said electrical signal comprises pulses having a repetition frequency being greater than or equal to 50 kHz and less than or equal to 10,000 kHz.

According to an aspect of the present invention, the repetition frequency is approximately one of 101 kHz, or 150 kHz, or 180 kHz, or 200 kHz.

Particularly, said nervous tissue is formed by the spinal cord. (i.e., the stimulation corresponds to a spinal cord stimulation (SCS), wherein the electrodes are adapted to apply said signal to the spinal cord.

According to an embodiment of the present invention, said device nervous tissue is configured for stimulation by at least one of the ganglia around the spinal cord, the brain, and/or the peripheral nerves.

Further, according to an embodiment of the device according to the present invention, the IPG allows programming of a ratio of the number of supra-threshold pulses to the number of sub-threshold pulses.

Further, according to an embodiment of the device according to the present invention, the IPG is configured to modulate said amplitudes of the pulses such that they oscillate, e.g. varies periodically.

Further, according to an embodiment of the device according to the present invention, the IPG is configured to modulate the amplitudes of the pulses so that they vary according to one of: a sinusoidal function, a square wave, burst of pulses, or a stimulus train.

Further, according to an embodiment of the device according to the present invention, the IPG is configured to modulate the amplitudes of the pulses such that the signal comprises (a) bursts comprising a plurality of supra-threshold pulses, as well as (b) a plurality of sub-threshold pulses in between each two or more successive bursts. The signal may also comprise (c) bursts comprising a plurality of sub-threshold pulses in between a single burst, and/or (d) bursts comprising a plurality of supra-threshold pulses in between a single sub-threshold pulse.

According to an aspect of the present invention, the frequency of supra-threshold pulses to the number of sub-threshold pulses is one of: fixed, selectable, or programmable, being greater than or equal to 100 kHz and less than or equal to 5,000 kHz.

According to an aspect of the invention, a method for stimulation of human or animal nervous tissue, particularly of the spinal cord, is disclosed, wherein an electrical signal (e.g. voltage) comprising pulses is applied to said tissue, wherein a repetition frequency of said pulses are greater than or equal to 51 kHz and less than or equal to 5,000 kHz, and wherein an amplitude of said pulses is modulated such that the electrical signal comprises sub-threshold pulses that do not elicit an action potential of the nervous tissue, and supra-threshold pulses that elicit an action potential of the nervous tissue.

Thus, particularly, sub-threshold stimuli/pulses are defined as delivering an electric charge via the electrode(s) without eliciting an action potential in the tissue, whereas supra-threshold stimuli are defined as delivering an electric charge via the electrode(s) with the intent or effect of eliciting an action potential. Particularly, the ratio between supra-threshold and sub-threshold stimuli/pulses is a function of amplitude modulation of the signal and the physiological system.

In other words, the present invention particularly describes a novel means to stimulate neurons or anatomical structures like the spinal cord, dorsal laminae, dorsal root ganglia (to name a few) to alleviate or relieve pain without inducing paresthesia (tingling, pins and needles sensation). This novel mechanism relies on delivering an amplitude modulated signal at frequencies of 100 kHz, 5,000 kHz. Recent evidence suggests that high frequency stimulation (1.5 kHz - 100 kHz) of the spinal cord may provide pain relief with the added benefit of being paresthesia free. The present invention permits the use of higher frequency stimulation (>100 kHz) by implementing amplitude modulation to the stimulation methodology.

Particularly, the ratio of sub-threshold to supra-threshold stimuli/pulses may be, but is not limited to, fixed or programmable ratios. The amplitudes may vary in a sinusoidal or square wave fashion, in bursts, pulses, or continuous trains. The amplitudes of stimuli/pulses may be fixed and programmable or may be oscillated or varied based on preprogrammed algorithms, or operator specifications.

According to a particular embodiment of the method according to the present invention, said nervous tissue is formed by the spinal cord, i.e. the stimulation corresponds to a spinal cord stimulation (SCS).

Further, according to an embodiment of the method according to the present invention, a ratio of the number of sub-threshold pulses to the number of supra-threshold pulses is fixed, or is selectable, or is programmable (i.e. can be adjusted by a person, e.g., the user or a medical practitioner).

Further, according to an embodiment of the method according to the present invention, the amplitudes are modulated such that the amplitudes oscillate between sub-threshold and supra-threshold pulses (e.g. vary with a fixed, selectable, or programmable periodicity).

Further, according to an embodiment of the method according to the present invention, the amplitudes of the pulses are modulated so that they vary according to one of: a sinusoidal (or similar) function, a square wave, or train of pulses which occur in bursts or individual pulses.

According to an embodiment of the method of the present invention, the amplitudes are modulated such that the signal comprises (a) bursts comprising a plurality of supra-threshold pulses, as well as (b) a plurality of sub-threshold pulses in between each two successive bursts.

According to yet another aspect of the present invention, a method for varying an electrical signal (e.g. voltage) is disclosed, which signal comprises pulses, wherein a repetition frequency of said pulses greater than or equal to 101 kHz and less than or equal to 5,000 kHz, and wherein a plurality of the amplitudes of said pulses is modulated such that the electrical signal comprises sub-threshold pulses that do not elicit an action potential of the nervous tissue when applied to said nervous tissue, and supra-threshold pulses that elicit an action potential of the nervous tissue when applied to said nervous tissue. This aspect of the present invention is merely concerned with the variation of the amplitudes, but not with an (e.g. SCS) in vivo stimulation for therapeutical purposes. This aspect may be further characterized by the individual features of the sub claims 2 to 6.

According to yet another aspect of the present invention, a device particularly for conducting the method according to the present invention is proposed.

The methods and devices disclosed herein allow for the use of higher frequency waveforms to produce paresthesia free therapy for pain relief. Additionally, neural priming and modulation of the duration of the refractory period may further be induced with the low amplitude component of the amplitude modulated signal thereby providing additional therapeutic benefit and energy cost savings compared to fixed amplitude high frequency stimulation.

Furthermore, detailed embodiments and features of the present invention will be described below with reference to the Figures, wherein
- Fig. 1: shows a device according to the present invention for providing nervous tissue stimulation, here as an example SCS;
- Fig. 2: shows a 200 kHz stimulation frequency amplitude modulation of a method/device according to the present invention;
- Fig. 3: shows a 160 kHz stimulation frequency with sine-wave modulation of amplitudes;
- Fig. 4: shows a 152 kHz stimulation frequency with oscillating modulation of amplitudes; and
- Fig. 5: shows a 300 kHz stimulation frequency with burst modulation of amplitudes.

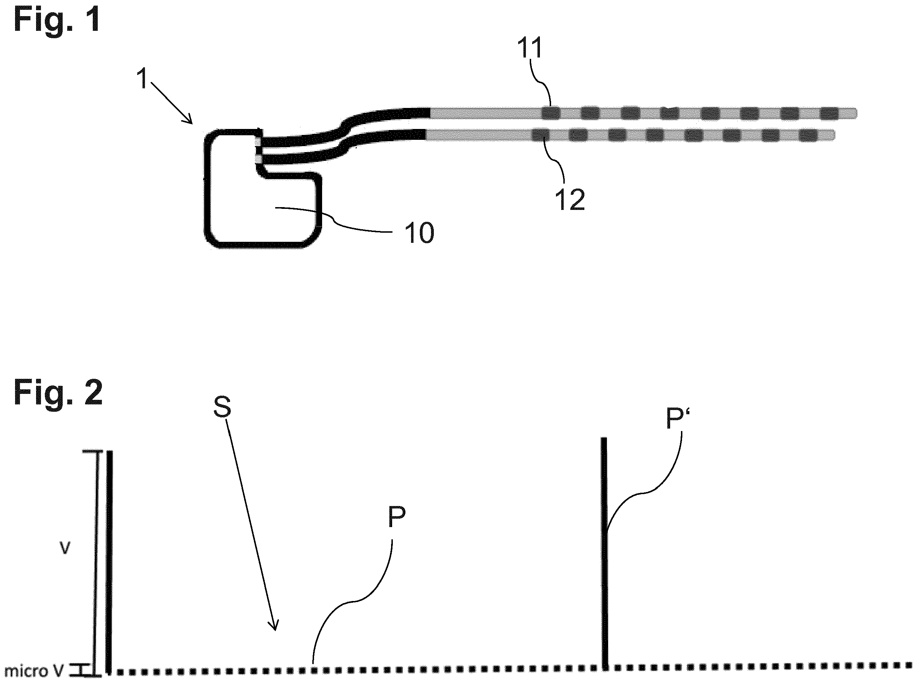

Utilizing a device 1 comprising an implantable pulse generator 10 (IPG) as shown in Fig. 1, a stimulation may be delivered to nervous tissue such as, but not limited to, the spinal cord or associated structures using a plurality of electrodes (such as those illustrated by 11, 12), wherein the frequency (pulse repetition rate) of stimulation is greater than or equal to 100 kHz. Sub-threshold stimuli pulses P as well as supra-threshold stimuli/pulses P' (cf. Figs. 2 to 5) are delivered to the target tissue. Particularly, sub-threshold stimuli or pulses P are defined as delivering an electric charge via the electrode(s) without eliciting an action potential in the tissue, whereas supra-threshold stimuli or pulses P' are defined as delivering an electric charge via the electrode(s) with the intent or effect of eliciting an action potential.

Particularly, the ratio between the number of supra-threshold and sub-threshold stimuli is a function of an amplitude modulation of the applied signal generated by the IPG 10.

The ratio of sub-threshold to supra-threshold stimuli or pulses P: P' per unit time may be, but is not limited to, fixed or programmable ratios. Further, the amplitudes of the pulses can be modulated such that they vary according to a sinusoidal or square wave function or can be modulated in bursts, trains, or pulses. The amplitudes of stimuli may be fixed or programmable or may be oscillated or varied through selectable or programmable methods, such as, but not limited to, algorithms.

Additional fixed, selectable, or programmable pulse widths may be part of the feature for sub-threshold and supra-threshold stimulation.

Additional fixed, selectable, or programmable quiescent period between pulses may be part of the feature for sub-threshold and supra-threshold stimulation.

Additional fixed, selectable, or programmable amongst a plurality of electrode stimulation modes may be used as part of this feature.

According to an embodiment shown in Fig. 2, the IPG 10 may deliver a programmed frequency of stimulation of 200 kHz (pulse repetition rate), wherein the ratio of supra-threshold (P') to sub-threshold pulses/(priming stimuli) P is 1:40. The amplitudes of the sub-threshold and supra-threshold pulses P, P' in this example may be fixed, selectable, or programmable. Here, an example is shown where the amplitudes of the sub-threshold pulses P is in the µV (Microvolt) range and the ones of the supra-threshold pulses P' are in the V (Volt) range. The resulting delivered signal S then shows the features shown in Fig. 2. However, the voltages delivered for P and P' are not limited to those provided in the example and may encompass a plurality of amplitudes between nanovolts (nV) to kilovolts (kV) inclusive.

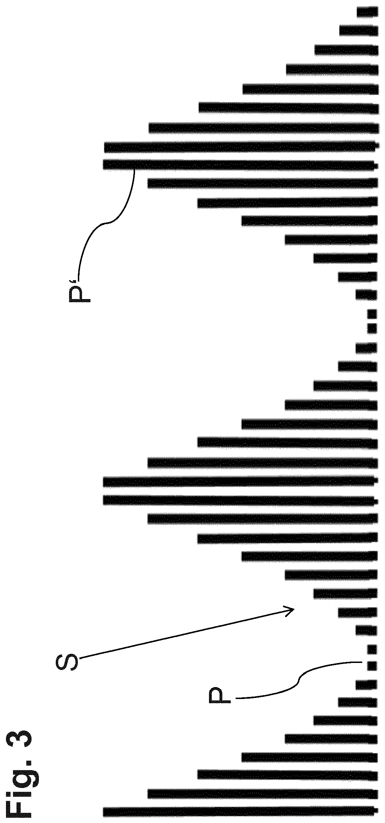

According to another example shown in Fig. 3, the programmed frequency (pulse repetition rate) of the stimulation may be 180 kHz, wherein the amplitudes of supra-threshold P' and sub-threshold (priming) stimuli P are modulated using, but not limited to, a sine-wave. The amplitude of the sub-threshold P and supra-threshold stimuli/pulses P' in this example may be a function of the sine-wave algorithm selected. Due to the modulation, one has a certain ratio of the number of supra-threshold pulses P' to the number of sub-threshold pulses P. Here, the resulting delivered signal S comprises the features shown in Fig. 3.

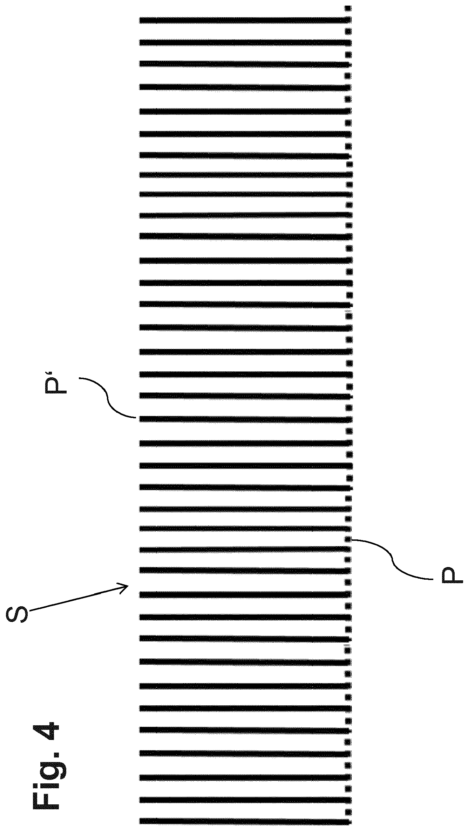

According to a further embodiment, the programmed frequency of stimulation can be (e.g. 152 kHz, pulse repetition frequency), wherein the ratio of supra-threshold (P') to sub-threshold pulses/(priming stimuli) P is defined by oscillating the amplitudes of the pulses P, P', or using a square wave. The amplitude of the sub-threshold and supra-threshold stimuli/pulses P, P' in this example can be a function of the system or a selection made by the operator. The resulting delivered signal S then shows the features shown in Fig. 4.

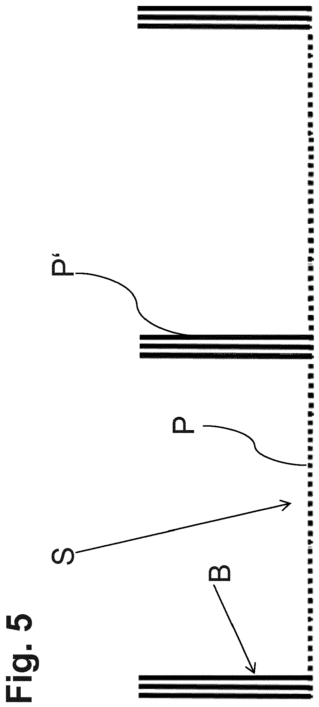

Furthermore, according to yet another embodiment, the frequency of stimulation may be programmed to amount to 300 kHz (pulse repetition frequency), wherein the ratio of supra-threshold (P') to sub-threshold pulses/(priming stimuli) P is defined using an amplitude dependent burst pattern. The amplitude of the sub-threshold and supra-threshold stimuli/pulses P, P' in this example can be a function of the fixed, selected, or programmed amplitudes. The resulting delivered signal S then shows the features shown in Fig. 5.

## Claims

1. Device for stimulation of nervous tissue, wherein the device (1) comprises an implantable pulse generator (10) that comprises electrodes (11, 12) and is configured to apply an electrical signal (S) via said electrodes (11, 12) to nervous tissue of a human or animal patient, such that said electrical signal (S) comprises pulses (P, P') having a repetition frequency being larger than 100kHz and smaller than 5,000 kHz, and wherein the implantable pulse generator (10) is further configured to modulate an amplitude of said pulses (P, P') such that the electrical signal (S) comprises sub-threshold pulses (P) that do not elicit an action potential, and supra-threshold pulses (P') that elicit an action potential.

2. Device according to claim 1, wherein the repetition frequency is approximately one of
101 kHz, or
150 kHz, or
180 kHz, or
200 kHz.

3. Device according to claim 1 or 2, **wherein** said device nervous tissue is configured for stimulation by at least one of
the spinal cord,
the ganglia around the spinal cord,
the brain,
and/or the peripheral nerves.

4. The device according to at least one of the claims 1 to 3, **wherein** a ratio of the number of supra-threshold pulses (P') to the number of sub-threshold pulses (P) is one of: fixed, selectable, or programmable.

5. The device according to at least one of the preceding claims, **wherein** the frequency of supra-threshold pulses (P') to the number of sub-threshold pulses (P) is one of: fixed, selectable, or programmable, being greater than or equal to 100 kHz and less than or equal to 5,000 kHz.

6. The device according to at least one of the preceding claims, **wherein** the amplitudes of the pulses (P, P') are modulated so that they vary according to one of: oscillation of the sub-threshold pulses (P) and supra-threshold pulses (P'), a sinusoidal function (or similar), a square wave, burst of pulses, or trains of pulses.
